(19) Europäisches Patentamt
European Patent Office
Office européen des brevets

(11) Veröffentlichungsnummer : **0 008 626**
**B1**

(12) # EUROPÄISCHE PATENTSCHRIFT

(45) Veröffentlichungstag der Patentschrift : **29.07.81**

(21) Anmeldenummer : **79102209.8**

(22) Anmeldetag : **02.07.79**

(51) Int. Cl.³ : **C 07 C 59/185**, C 07 C 59/19, C 07 C 59/347, C 07 C 65/32, C 07 C 65/21, C 09 D 3/58, C 09 D 3/72

(54) **Ammoniumsalze von Alpha-Ketocarbonsäuren, Ihre Verwendung zur Herstellung von Aminen und sie enthaltende Beschichtungsmassen.**

(30) Priorität : **14.07.78 DE 2830953**

(43) Veröffentlichungstag der Anmeldung : **19.03.80 (Patentblatt 80/06)**

(45) Bekanntmachung des Hinweises auf die Patenterteilung : **29.07.81 Patentblatt 81/30**

(84) Benannte Vertragsstaaten : **BE DE FR GB IT NL**

(56) Entgegenhaltungen :
**DE - A 1 - 2 357 859**
**FR - M - 1 642**
**US - A - 3 338 804**
**CHEMICAL ABSTRACTS, Band 48, Nr. 15, 10. August 1954, Columbus, Ohio, U.S.A.**
**P. SIMONART et al. : "The amino-acid metabolism in Aspergillus oryzae. III. Free amino acids in the mycelium grown on different carbon sources in presence of ammonia".**

(73) Patentinhaber : **BAYER AG**
**Zentralbereich Patente, Marken und Lizenzen**
**D-5090 Leverkusen 1, Bayerwerk (DE)**

(72) Erfinder : **Mayer, Wolfram, Dr.**
**Mozartstrasse 10**
**D-4150 Krefeld 1 (DE)**
Erfinder : **Rudolph, Hans, Dr.**
**Haydnstrasse 9**
**D-4150 Krefeld 1 (DE)**
Erfinder : **de Cleur, Eckhard, Dr.**
**Aubruchsgraben 14**
**D-4100 Duisburg 46 (DE)**
Erfinder : **Schönfelder, Manfred, Dr.**
**Höhenstrasse 126**
**D-5090 Leverkusen 3 (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Imprimerie Jouve, 17, rue du Louvre, 75001 Paris, France

EP 0 008 626 B1

# Ammoniumsalze von Alpha-Ketocarbonsäuren, ihre Verwendung zur Herstellung von Aminen und sie enthaltende Beschichtungsmassen

Die vorliegende Erfindung betrifft neue Ammoniumsalze von α-Ketocarbonsäuren, ihre Verwendung zur in situ — Herstellung von Aminen durch photochemische Zersetzung sowie photochemisch härtbare Beschichtungsmassen, die derartige Ammoniumsalze enthalten.

Mono- und Polyamine werden auf Grund ihrer sehr vielseitigen chemisch-physikalischen Eigenschaften als Reaktionspartner bei den unterschiedlichsten chemischen Umsetzungen eingesetzt. Erwähnt seien hier nur die auf ihre Basizität zurückzuführende katalytische Wirkung für zahlreiche chemische Reaktionen oder ihre Verwendung als Vernetzungsmittel für Polyurethan- und Epoxidharze. Für viele Anwendungswecke ist es erwünscht, nicht die freien Amine einzusetzen, sondern die Amine dem Reaktionsgemisch in einer verkappten Form zuzusetzen, aus welcher sie zu einem definierten Zeitpunkt freigesetzt werden, wodurch die gewünschte Reaktion gestartet wird.

So ist es z.B. möglich, aus Enaminen, Ketiminen und Aldiminen Amine durch Zufügen von Wasser zu erhalten. Nachteilig ist jedoch die große Feuchtigkeitsempfindlichkeit dieser Verbindungen ; tertiäre Aminstickstoffatome können auf diese Weise nicht blockiert werden. Es ist weiterhin bekannt ; daß sich aus quartären Ammoniumsalzen durch Erhitzen auf über 100 °C Amine in Freiheit setzen lassen (Houben-Weyl, Methoden der Organischen Chemie, Bd. XI, 1). In vielen Fällen ist es jedoch erwünscht, die Amine schon bei wesentlich niedrigeren Temperaturen zu deblockieren.

Überraschenderweise wurde nunmehr gefunden, daß aus Ammoniumsalzen von α-Ketocarbonsäuren die Amine auch bei Raumtemperatur unter der Einwirkung von Licht der Wellenlänge 250-500 nm unter Decarboxylierung der Ketocarbonsäure freigesetzt werden.

Gegenstand der vorliegenden Erfindung sind daher Ammoniumsalze auf Basis von Aminen eines zwischen 31 und 500 liegenden Molekulargewichts und von α-Ketocarbonsäuren der allgemeinen Formel

$$A—CO—COOH \qquad (I)$$

in welcher

A für Wasserstoff, eine Hydroxyl- oder Carboxylgruppe, —COR, —CN, einen gegebenenfalls verzweigten Alkylrest mit 1 bis 6 C-Atomen, welcher gegebenenfalls durch Halogen, —OH, —COOH, —COOR, —CN, —OR, —COR oder —COR′ substituiert ist, einen Cycloalkylrest mit 4 bis 10 C-Atomen, einen Aryl- oder Aralkylrest mit 6 bis 15 C-Atomen, welcher gegebenenfalls durch —OH, —R, OR, —SR, Halogen, $—NO_2$, —COR, —COOH, —CN, —COOR, $—CONH_2$, —OR′, —SR′ oder —COR′ substituiert ist, oder einen Sauerstoff und/oder Stickstoff als Heteroatom enthaltenden heterocyclischen Rest mit 4 bis 10 C-Atomen darstellt, wobei

R für eine gegebenenfalls halogensubstituierte Alkylgruppe mit 1 bis 6 C-Atomen und

R′ für eine Arylgruppe mit 6 bis 12 C-Atomen stehen,

wobei das Salz auf Basis von Triäthanolamin und Brenztraubensäure ausgenommen ist.

In der vorveröffentlichten Literatur sind bereits einige Ammoniumsalze von β-Ketocarbonsäuren beschrieben. Diese vorbeschriebenen Salze sind jedoch mit den erfindungsgemäßen Salzen nicht identisch, außerdem fehlt in den Vorveröffentlichungen jeglicher Hinweis auf die Eignung der vorbeschriebenen Salze als blockierte Amine, aus welchen die freien Amine durch Einwirkung von Licht und/oder Hitze erhalten werden können. So beschreibt beispielsweise FR-M-16 42 ein bestimmtes Betain, welches als Arzneimittel verwendbar ist. Die US-A-3 338 804 erwähnt das am Stickstoff unsubstituierte Ammoniumsalz der Brenztraubensäure und das Ammoniumsalz aus Brenztraubensäure und Triäthanolamin. Die in dieser Vorveröffentlichung genannten Salze dienen als Zusatzmittel in wäßrigen Metallsalzlösungen, die zur Elektrotauchlackierung von Metallen verwendbar sind. In Chemical Abstracts, Band 48, Nr. 15, Spalte 88 73a wird ferner die Rolle von am Stickstoff unsubstituerten Ammoniumsalzen der Brenztraubensäure oder der α-Ketoglutarsäure bei bestimmten Stoffwechselvorgängen diskutiert. Schließlich erwähnt Beilstein, Handbuch der organischen Chemie, 4. Auflage, 2. Ergänzungswerk, Band 10, Seite 454, das am Stickstoff unsubstituierte Ammoniumsalz der Phenylglyoxylsäure ohne jeglichen Hinweis auf die Möglichkeit der Freisetzung des dem Salz zugrundeliegenden Amins durch Bestrahlung und/oder Einwirkung von Hitze.

Beispiele für erfindungsgemäß geeignete Säuren der allgemeinen Formel (I) sind Glyoxylsäure, Brenztraubensäure (die jedoch nicht in Kombination mit Triäthanolamin zur Anwendung gelangen soll), Cyclobutylglyoxylsäure Cyclopentylglyoxylsäure, Cyclohexylglyoxylsäure, Cyclohexenylglyoxylsäure, Trichlorbrenztraubensäure, Methoxybrenztrauben, säure, Acetylbrenztraubensäure, Oxalsäure, Mesoxalsäure, Oxalessigsäure, Methyloxalessigsäure, Phenylbrenztraubensäure, Benzylbrenztraubensäure, Benzoylbrenztraubensäure, p-Chlorphenylbrenztraubensäure, Naphthoylbrenztraubensäure, Diäthoxybrenztraubensäure, Furylbrenztraubensäure, Pyridinbrenztraubensäure, Mercaptobrenztraubensäure, Phenylglyoxylsäure, p-Chlorphenylglyoxylsäure, p-Methoxyphenylglyoxylsäure, p-Nitrophenylglyoxylsäure, Naphthylglyoxylsäure, Anthracenglyoxylsäure, Azulenglyoxylsäure, Benzimidazolglyoxylsäure, Benzofuranglyoxylsäure, Benzoxazinglyoxylsäure, Furylglyoxylsäure, Pyridinglyoxylsäure, Chinolinglyoxylsäure und Thiophenglyoxylsäure.

Pyridinglyoxylsäure, Chinolinglyoxylsäure und Thiophenglyoxylsäure.

Erfindungsgemäß bevorzugt sind Oxalsäure, Brenztraubensäure, Phenylbrenztraubensäure sowie insbesondere Phenylglyoxylsäure, am aromatischen Ring durch $C_1$-$C_3$-Alkyl-, $C_1$-$C_3$-Alkoxy-, Hydroxy- oder Nitrogruppen, Halogen oder einen Phenylrest substituierte Phenylglyoxylsäure und auch α-Naphthylglyoxylsäure.

Als Aminkomponente kommen für die erfindungsgemäßen Ammoniumsalze beliebige, ein oder mehrere, primäre und/oder sekundäre und/oder tertiäre (bevorzugt tertiäre) Aminstickstoffatome enthaltende Verbindungen mit einem Molekulargewicht zwischen 31 und 500, vorzugsweise zwischen 100 und 300, in Frage. Diese Verbindungen enthalten vorzugsweise 1 bis 5, besonders bevorzugt 1 bis 3, Aminstickstoffatome und können daneben noch andere funktionelle Gruppen wie z.B. Hydroxyl-, Mercapto-, Äther-, Thioäther-, Amid- und Estergruppen oder auch Halogenatome aufweisen.

Beispiele für erfindungsgemäß in Frage kommende Amine sind Methylamin, Dimethylamin, Äthylamin, Diäthylamin, Äthanolamin, Diäthanolamin; tertiäre Amine, wie Triäthylamin, Tributylamin, N-Methylmorpholin, N-Äthyl-morpholin, N,N,N',N'-Tetramethyl-äthylendiamin, Pentamethyl-diäthylentriamin und höhere Homologe (DE-Offenlegungsschriften 2 624 527 und 2 624 528), 1,4-Diazabicyclo-(2,2,2)-octan, N-methyl-N'-dimethylaminoäthylpiperazin, Bis-(dimethylaminoalkyl)-piperazine (DE-Offenlegungsschrift 2 636 787), N,N-Dimethylbenzylamin, N,N-Dimethylcyclohexylamin, N,N-Diäthylbenzylamin, Bis-(N,N-diäthylaminoäthyl)-adipat, N,N,N',N'-Tetramethyl-1,3-butandiamin, N,N-Dimethyl-β-phenyläthylamin, 1,2-Dimethylimidazol, 2-Methylimidazol, monocyclisches und bicyclische Amidine (DE-Offenlegungsschrift 1 720 633), Bis-(dialkylamino) alkyl-äther (US-Patentschrift 3 330 782, DE-Auslegeschrift 1 030 558, DE-Offenlegungsschriften 1 804 361 und 2 618 280) sowie Amidgruppen (vorzugsweise Formamidgruppen) aufweisende tertiäre Amine gemäß den DE-Offenlegungsschriften 2 523 633 und 2 732 929); aktive Wasserstoffatome aufweisende tertiäre Amine, z.B. Triäthanolamin, (welches jedoch nicht in Kombination mit Brenztraubensäure zur Anwendung gelangen soll), Triisopropanolamin, N-Methyl-diäthanolamin, N-Äthyldiäthanolamin, N,N-Dimethyl-äthanolamin, deren Umsetzungsprodukte mit Alkylenoxiden wie Propylenoxid und/oder Äthylenoxid sowie sekundär-tertiäre Amine gemäß DE-Offenlegungsschrift 2 732 292; ferner Silaamine mit Kohlenstoff-Silizium-Bindungen, wie sie z.B. in der DE-Patentschrift 1 229 290 (entsprechend der US-Patentschrift 3 620 984) beschrieben sind, z.B. 2,2,4-Trimethyl-2-silamorpholin und 1,3-Diäthylaminomethyl-tetramethyldisiloxan.

Erfindungsgemäß geeignete primäre aliphatische Diamine sind beispielsweise Äthylendiamin, 1,4-Tetramethylendiamin, 1,11-Undecamethylendiamin, 1,12-Dodecamethylendiamin sowie deren Gemische, 1-Amino-3,3,5-trimethyl-5-aminomethylcyclohexan (« Isophorondiamin »), 2,4- und 2,6-Hexahydrotoluylendiamin sowie deren Gemische, Perhydro-2,4'- und 4,4'-diaminodiphenylmethan, p-Xylylendiamin, Bis-(3-aminopropyl)-methylamin, Diamino-perhydroanthrazene (DE-Offenlegungsschrift 2 638 731) und cycloaliphatische Triamine gemäß DE-Offenlegungsschrift 2 614 244. Auch Hydrazin und substituierte Hydrazine, z.B. Methylhydrazin, N,N'-Dimethylhydrazin und deren Homologe sowie Säuredihydrazide kommen erfindungsgemäß in Betracht, z.B. Carbodihydrazid, Oxalsäuredihydrazid, die Dihydrazide von Malonsäure, Bernsteinsäure, Glutarsäure, Adipinsäure, β-Methyladipinsäure, Sebazinsäure, hydracrylsäure und Terephthalsäure; Semicarbazido-alkylen-hydrazide wie z.B. β-Semicarbazidopropionsäurehydrazid (DE-Offenlegungsschrift 1 770 591), Semicarbazido-alkylencarbazinester wie z.B. 2-Semicarbazidoäthyl-carbazinester (DE-Offenlegungsschrift 1 918 504) oder auch Amino-semicarbazid-Verbindungen wie z.B. β-Aminoäthyl-semicarbazido-carbonat (DE-Offenlegungsschrift 1 902 931).

Als Beispiele für primäre aromatische Diamine seien Bis-anthranilsäureester gemäß den DE-Offenlegungsschriften 2 040 644 und 2 160 590, 3,5- und 2,4-Diaminobenzoesäureester gemäß DE-Offenlegungsschrift 2 025 900, die in den DE-Offenlegungsschriften 1 803 635 (US-Patentschriften 3 681 290 und 3 736 350), 2 040 650 und 2 160 589 beschriebenen estergruppenhaltigen Diamine, die Äthergruppen aufweisenden Diamine gemäß De-Offenlegungsschriften 1 770 525 und 1 809 172 (US-Patentschriften 3 654 364 und 3 736 295), gegebenenfalls in 5-Stellung substituierte 2-Halogen-1,3-Phenylendiamine (DE-Offenlegungschriften 2 001 772, 2 025 896 und 2 065 869), 3,3'-Dichlor-4,4'-diamino-diphenylmethan, Toluylendiamin, 4,4'-Diaminodiphenylmethan, 4,4'-Diaminodiphenyldisulfide (DE-Offenlegungschrift 2 404 976), Diaminodiphenyldithioäther (DE-Offenlegungsschrift 2 509 404), durch Alkylthiogruppen substituierte aromatische Diamine (DE-Offenlegungsschrift 2 638 760), Diaminobenzolphosphonsäureester (DE-Offenlegungsschrift 2 459 491), Sulfonat- oder Carboxylatgruppen enthaltende aromatische Diamine (DE-Offenlegungsschrift 2 720 166) sowie die in der DE-Offenlegungsschrift 2 635 400 aufgeführten hochschmelzenden Diamine genannt. Beispiele für aliphatisch-aromatische Diamine sind die Aminoalkylthioaniline gemäß DE-Offenlegungsschrift 2 734 574.

Als erfindungsgemäß geeignete Monoamine seien noch Butyl- und Dibutylamin, Octylamin, Stearylamin, N-Methylstearylamin, Pyrrolidin, Piperidin und Cyclohexylamin genannt.

Erfindungsgemäß bevorzugte Amine sind tert.-Butylamin, Diäthylamin, Diäthanolamin, Triäthylamin, Dibutylamin, Tributylamin, N,N-Dimethylbenzylamin, Diazabicyclooctan (DABCO) und Diazabicycloundecen (DBU).

Aus der Literatur ist bekannt, daß gewisse α-Ketocarbonsäuren in Gegenwart von Aminen beim Erhitzen zersetzt werden. (J. C. Craig und L. R. Kray in J. Org. Chem. *31,* 871 (1968); S. D. Paul und G. D. Pradham, Indian J. Chem. *9,* 318 (1971)). So erhält man z.B. aus Phenylglyoxylsäure-$d_1$ beim Erhitzen auf 130 °C in Gegenwart von N-Äthylmorpholin Benzaldehyd-$d_1$.

Die erfindungsgemäßen Ammoniumsalze sind im allgemeinen kristalline Verbindungen mit definiertem Schmelzpunkt. Sie sind in vielen organischen Lösungsmitteln löslich, z.B. in Aceton, Acetonitril, Chloroform, Methylenchlorid, Methanol, Äthanol und Äthylenglykolmonomethylätheracetat.

Die erfindungsgemäßen Ammoniumsalze können in einfacher Weise hergestellt werden, indem man die α-Ketocarbonsäuren der allgemeinen Formel I bei Temperaturen von − 20 bis + 50 °C, vorzugsweise bei Raumtemperatur, gegebenenfalls in Anwesenheit eines organischen Lösungsmittels wie Äther, Benzol, Toluol, Chlorbenzol, Petroläther, Aceton, Dioxan, Chloroform etc., mit der Aminkomponente umsetzt. Im allgemeinen wird dabei pro Aminäquivalent etwa 1 Mol der Ketocarbonsäure eingesetzt. Bei vielen Diaminen (z.B. Äthylendiamin oder Diazabicyclooctan) sind auch die Salze mit nur 1 Äquivalent an α-Ketocarbonsäure (also die Monoammoniumsalze) als erfindungsgemäße latente Initiatoren brauchbar. Auch solche nur teilweise mit α-Ketocarbonsäuren der Formel I neutralisierte Polyamine werden daher von der vorliegenden Erfindung mitumfaßt. Im übrigen läßt sich durch einen einfachen Vorversuch feststellen, wieviele Aminstickstoffatome eines Polyamins in die Ammoniumsalzform übergeführt werden müssen, um die gewünschte Desaktivierung zu erreichen.

Wie schon erwähnt, können aus den erfindungsgemäßen Ammoniumsalzen unter photochemischer Spaltung die zugrundeliegenden Amine zu jedem gewünschten Zeitpunkt unter sehr milden Bedingungen (schon bei Raumtemperatur) durch Bestrahlen mit Licht einer Wellenlänge zwischen 250 und 500 nm, vorzugsweise 300-400 nm in Freiheit gesetzt werden.

Gegenstand der Erfindung ist somit auch die Verwendung der erfindungsgemäßen Ammoniumsalze zur Herstellung der ihnen zugrundeliegenden Amine, dadurch gekennzeichnet, daß man die Ammoniumsalze mit Licht einer Wellenlänge zwischen 250 und 500 nm bestrahlt und/oder auf 50-130°, vorzugsweise 50-80 °C erhitzt.

Es ist erfindungsgemäß vorteilhaft, die Photoreaktion durch Zusatz von an sich bekannten Triplett-Sensibilisatoren wie z.B. Benzophenon, Acetophenon, Propiophenon, Xanthon, Thioxanthon oder Triphenylen zu sensibilisieren. Diese Sensibilisatoren werden vorzugsweise in einer Menge von 0,1 bis 30 Gew.-%, besonders bevorzugt 3 bis 10 Gew.-%, bezogen auf Ammoniumsalz, zugegeben.

Die erfindungsgemäßen Ammoniumsalze sind insbesondere als durch UV-Strahlen aktivierbare Härtungskatalysatoren für Harze oder Harzkompositionen geeignet. Solche Harze sind z.B. Epoxyharze oder Lacke auf Basis von Isocyanaten, die entweder als solche, in chemisch modifizierter Form oder in Mischung mit Harzen anderer Art verwendet werden.

Nach dem Isocyanat-Polyadditions-Verfahren aus Polyhydroxylverbindungen und Polyisocyanaten erhaltene Lacküberzüge mit vernetzter hochmolekularer Polyurethanstruktur sind bekannt. Diese technisch wertvollen Überzüge lassen sich sowohl von Hand als auch maschinell, z.B. durch Spritzen, Tauchen oder Gießen, auftragen. In der Praxis wird entweder das sogenannte zweikomponenten- oder das Einkomponenten-Verfahren angewandt. Im ersteren Fall werden die beiden Komponenten (Polyisocyanat einerseits ; gegenüber Isocyanaten reaktive Verbindung andererseits), gegebenenfalls in Anwesenheit eines Lösungsmittels, vermischt. Eine Reaktion der beiden Komponenten läßt sich dabei nur dann vollständig vermeiden, wenn man verkappte Polyisocyanate einsetzt, die erst beim Erhitzen das freie Isocyanat abspalten ; die Anwendung solcher Systeme ist aber auf einzubrennende Lackierungen beschränkt. Immerhin haben auch die Lackmischungen, welche freie Polyisocyanate enthalten, eine mehr oder weniger lange Lebensdauer (Pot-life), die es ermöglicht, diese Lacke in technisch befriedigender Weise von Hand aus oder maschinell auf die Unterlagen aufzutragen, auf denen sie dann unter Polyurethanbildung endgültig aushärten und vernetzen. Einkomponentensysteme enthalten dagegen ein Addukt mit freien Isocyanatgruppen aus Polyhydroxylverbindung und einem Überschuß an Polyisocyanat, wobei nach dem Auftrag durch Reaktion der freien NCO-Gruppen im Lack mit Wasser (Luftfeuchtigkeit) Vernetzung erfolgt. Auch hierbei muß man dafür Sorge tragen, daß eine vorzeitige Vernetzung während der Lagerung des Lackes unterbleibt, indem man die Luftfeuchtigkeit ausschließt und/oder wasserbeseitigende Mittel zusetzt.

Andererseits ist es erwünscht, daß nach dem Auftragen des Lackes auf die Unterlage eine möglichst schnelle Vernetzung und Trocknung erfolgt. Sowohl bei Einkomponentenals auch bei Zweikomponentenlacken ist es möglich, durch den Zusatz von an sich bekannten Reaktionsbeschleunigern die Härtungsreaktion zu fördern. Die Forderung der beschleunigten Vernetzungsreaktion auf der Unterlage widerspricht aber der gleichzeitig zu erhebenden Forderung nach einer möglichst langen Verarbeitungsfähigkeit. Im Prinzip wäre es denkbar, der Lackmischung einen Katalysator erst kurz vor der Applikation auf die Unterlage zuzufügen. Das mag zwar bei einem Handverfahren im kleinen Maßstab möglich sein, verbietet sich aber bei großtechnischer, maschineller Arbeitsweise, da die Lackmischungen in der Maschine längere Verweilzeiten, zum Teil bei höherer Temperatur, besitzen und eine ungewollte Verkürzung der Verarbeitungsspanne (Pot-life) durch die beschleunigende Wirkung des Katalysators nicht zu vermeiden ist. Man hat auch bereits daran gedacht, den Katalysator noch nachträglich nach dem Auftragen der Lackmischung auf die Unterlage, etwa durch Aufdüsen im gasförmigen Zustand, aufzubringen ; das aber erfordert aufwendige zusätzliche maschinelle Einrichtungen. Überdies sind zwangsläufig nur einige wenige Katalysatoren auf diese Weise verarbeitbar.

Aus der DE-A-1 621 883 ist es bekannt, auf die zu lackierende Oberfläche zunächst eine Lackschicht aus einem physikalisch trocknenden, einen für Isocyanat-Polyadditions-Reaktionen an sich bekannten Katalysator enthaltenden Bindemittel und anschließend einen katalysatorfreien Polyurethanlack aufzubringen. Auf diese Weise gelingt es, ohne Beeinträchtigung der Lagerstabilität und der Verarbeitungsfähigkeit einer Polyurethanlackmischung die Trocknungszeit des Lacks erheblich herabzusetzen. Nachteilig ist dabei aber der zusätzliche Aufwand der zweiten Lackierung.

Prinzipiell dieselben Probleme wie bei den oben geschilderten Polyurethan-Einkomponenten- und Zweikomponentensystemen treten auch bei Beschichtungsmassen auf Basis von Epoxyharzen auf, deren Vernetzung im allgemeinen ebenfalls mittels tertiärer Amine katalysiert werden muß. In der DE-A-2 357 859 wird vorgeschlagen, zu diesem Zweck den Beschichtungsmassen Salze von tertiären Aminen mit bestimmten α-substituierten Carbonsäuren zuzusetzen. Beim Erhitzen auf 70 bis 200 °C zersetzen sich diese Ammoniumsalze unter Decarboxylierung, wonach unter dem katalytischen Einfluß des freiwerdenden Amins die Beschichtung rasch aushärtet.

Mit den erfindungsgemäßen Ammoniumsalzen werden nunmehr verkappte tertiäre Aminkatalysatoren zur Verfügung gestellt, welche eine rasche Aushärtung der Beschichtung schon bei Raumtemperatur durch Bestrahlen mit kurzwelligem Licht ermöglichen und welche Lackierungen mit besonders glänzender Oberfläche ergeben. Andererseits können aus den erfindungsgemäßen Ammoniumsalzen die Amine auch durch Erwärmen auf ca. 50-130 °C in Freiheit gesetzt werden.

Gegenstand der vorliegenden Erfindung sind somit auch Beschichtungsmassen auf Basis von in Gegenwart von Aminen aushärtenden Polyurethan- oder Epoxyharzvorprodukten, welche dadurch gekennzeichnet sind, daß sie 0,1 bis 40 Gew.-%, vorzugsweise 0,3 bis 15 Gew.-%, bezogen auf Feststoff, der erfindungsgemäßen Ammoniumsalze sowie gegebenenfalls Sensibilisatoren enthalten.

Als Grundlage für die erfindungsgemäßen Beschichtungsmassen kommen die in Gegenwart von Aminen vernetzenden Einkomponenten- und Zweikomponentenpolyurethansysteme in Betracht, wie sie in der Lack- und Beschichtungstechnik an sich bekannt sind. Einkomponentensysteme sind, wie bereits oben kurz erwähnt, gegebenenfalls in inerten organischen Lösungsmitteln gelöste Vorpolymerisate mit einem Gehalt von ca. 1 bis 25 Gew.-%, vorzugsweise 2,5 bis 19 Gew.-%, an freien NCO-Gruppen, welche durch Umsetzung von höhermolekularen und/oder niedermolekularen Verbindungen mit gegenüber Isocyanaten reaktiven Gruppen, wie sie nachstehend beschrieben werden, mit einem Überschuß an den nachstehend beschriebenen Polyisocyanaten hergestellt wurden. Bei Zweikomponentenpolyurethanen handelt es sich um ein gegebenenfalls in einem inerten organischen Lösungsmittel gelöstes Gemisch aus einer höhermolekularen Polyhydroxylverbindung, (z.B. einem Hydroxylgruppen aufweisenden Vorpolymeren aus Polyisocyanaten und einem Überschuß an Polyolen) einerseits und einem Polyisocyanat andererseits.

Für die erfindungsgemäßen Beschichtungsmassen kommen aliphatische, cycloaliphatische, araliphatische, aromatische und heterocyclische Polyisocyanate in Frage, wie sie z.B. von W. Siefken in Justus Liebigs Annalen der Chemie, 562, Seiten 75 bis 136, beschrieben werden, beispielsweise solche der Formel

$$Q\,(NCO)_n$$

in der n = 2-4, vorzugsweise 2, und Q einen aliphatischen Kohlenwasserstoffrest mit 2-18, vorzugsweise 6-10 C-Atomen, einen cycloaliphatischen Kohlenwasserstoffrest mit 4-15, vorzugsweise 5-10 C-Atomen, einen aromatischen Kohlenwasserstoffrest mit 6-15, vorzugsweise 6-13 C-Atomen, oder einen araliphatischen Kohlenwasserstoffrest mit 8-15, vorzugsweise 8-13 C-Atomen, bedeuten, z.B. Äthylen-diisocyanat, 1,4-Tetramethylendiisocyanat, 1,6-Hexamethylendiisocyanat, Trimethylhexamethylendiisocyanat, 1,12-Dodecandiisocyanat, Cyclobutan-1,3-diisocyanat, Cyclohexan-1,3- und -1,4-diisocyanat sowie beliebige Gemische dieser Isomeren, 1-Isocyanato-3,3,5-trimethyl-5-isocyanatomethyl-cyclohexan (DE-Auslegeschrift 1 202 785, US-Patentschrift 3 401 190), 2,4- und 2,6-Hexahydrotoluylendiisocyanat sowie beliebige Gemische dieser Isomeren, Hexahydro-1,3- und/oder -1,4-phenylendiisocyanat, Perhydro-2,4'- und/oder -4,4'-diphenylmethan-diisocyanat, 1,3- und 1,4-Phenylendiisocyanat, 2,4- und 2,6-Toluylendiisocyanat sowie beliebige Gemische dieser Isomeren, Diphenylmethan-2,4'- und/oder -4,4'-diisocyanat sowie Naphthylen-1,5-diisocyanat.

Ferner kommen beispielsweise erfindungsgemäß in Frage: Triphenylmethan-4,4',4''-triisocyanat, Polyphenyl-polymethylenpolyisocyanate, wie sie durch Anilin-Formaldehyd-Kondensation und anschließende Phosgenierung erhalten und z.B. in den GB-Patentschriften 874 430 und 848 671 beschrieben werden, m- und p-Isocyanatophenylsulfonyl-isocyanate gemäß der US-Patentschrift 3 454 606, perchlorierte Arylpolyisocyanate, wie sie z.B. in der DE-Auslegeschrift 1 157 601 (US-Patentschrift 3 277 138) beschrieben werden, Carbodiimidgruppen aufweisende Polyisocyanate, wie sie in der DE-Patentschrift 1 092 007 (US-Patentschrift 3 152 162) sowie in den DE-Offenlegungsschriften 2 504 400, 2 537 685 und 2 552 350 beschrieben werden, Norbornan-Diisocyanate gemäß US-Patentschrift 3 492 330, Allophanatgruppen aufweisende Polyisocyanate, wie sie z.B. in der GB-Patentschrift 994 890, der BE-Patentschrift 761 626 und der NL-Patentanmeldung 7 102 524 beschrieben werden, Isocyanuratgruppen aufweisende Polyisocyanate, wie sie z.B. in der US-Patentschrift 3 001 973, in den DE-Patentschriften 1 022 789, 1 222 067 und 1 027 394 sowie in den DE-Offenlegungsschriften 1 929 034 und 2 004 048 beschrieben werden, Urethangruppen aufweisende Polyisocyanate, wie sie z.B. in der BE-Patentschrift 752 261 oder in den US-Patentschriften 3 394 164 und 3 644 457 beschrieben werden, acylierte Harnstoffgruppen aufweisende Polyisocyanate gemäß der DE-Patentschrift 1 230 778, Biuretgruppen aufweisende Polyisocyanate, wie sie z.B. in den US-Patentschriften 3 124 605, 3 201 372 und 3 124 605 sowie in der GB-Patentschrift

889 050 beschrieben werden, durch Telomerisationsreaktionen hergestellte Polyisocyanate, wie sie z.B. in der US-Patentschrift 3 654 106 beschrieben werden, Estergruppen aufweisende Polyisocyanate, wie sie z.B. in den GB-Patentschriften 965 474 und 1 072 956, in der US-Patentschrift 3 567 763 und in der DE-Patentschrift 1 231 688 genannt werden, Umsetzungsprodukte der obengenannten Isocyanate mit Acetalen gemäß der DE-Patentschrift 1 072 385 und polymere Fettsäureester enthaltende Polyisocyanate gemäß der US-Patentschrift 3 455 883.

Es ist auch möglich, die bei der technischen Isocyanatherstellung anfallenden, Isocyanatgruppen aufweisenden Destillationsrückstände, gegebenenfalls gelöst in einem oder mehreren der vorgenannten Polyisocyanate, einzusetzen. Ferner ist es möglich, beliebige Mischungen der vorgenannten Polyisocyanate zu verwenden.

Bevorzugte Polyisocyanate sind Hexamethylendiisocyanat, dessen Isocyanurat und dessen Biuret ; 1-Isocyanato-3,3,5-trimethyl-5-isocyanatomethyl-cyclohexan (Isophorondiisocyanat) ; die Toluylendiisocyanate und deren Isocyanurate ; das Mischisocyanurat aus Toluylendiisocyanat und Hexamethylendiisocyanat ; das Umsetzungsprodukt aus 1 Mol Trimethylolpropan und 3 Mol Toluylendiisocyanat sowie rohes Diphenylmethandiisocyanat.

Geeignete höhermolekulare Verbindungen mit mindestens zwei gegenüber Isocyanaten reaktionsfähigen Wasserstoffatomen sind solche mit einem Molekulargewicht in der Regel von 400 bis 50 000. Hierunter versteht man neben Aminogruppen, Thiolgruppen oder Carboxylgruppen aufweisenden Verbindungen vorzugsweise Hydroxylgruppen aufweisende Verbindungen, insbesondere zwei bis acht Hydroxylgruppen aufweisende Verbindungen, speziell solche vom Molekulargewicht 500 bis 25 000, vorzugsweise 700 bis 20 000, z.B. mindestens zwei, in der Regel 2 bis 8, vorzugsweise aber 2 bis 4, Hydroxylgruppen aufweisende Polyester, Polyäther, Polythioäther, Polyacetale, Polycarbonate, Polyesteramide und OH-Präpolymere, wie sie für die Herstellung von homogenen und von zellförmigen Polyurethanen an sich bekannt sind :

a) Die in Frage kommenden Hydroxylgruppen aufweisenden Polyester sind z.B. Umsetzungsprodukte von mehrwertigen, vorzugsweise zweiwertigen und gegebenenfalls zusätzlich dreiwertigen Alkoholen mit mehrwertigen, vorzugsweise zweiwertigen, Carbonsäuren. Anstelle der freien Polycarbonsäuren können auch die entsprechenden Polycarbonsäureanhydride oder entsprechende Polycarbonsäureester von niedrigen Alkoholen oder deren Gemische zur Herstellung der Polyester verwendet werden. Die Polycarbonsäuren können aliphatischer, cycloaliphatischer, aromatischer und/oder heterocyclischer Natur sein und gegebenenfalls, z.B. durch Halogenatome, substituiert und/oder ungesättigt sein.

Als Beispiele für solche Carbonsäuren und deren Derivate seien genannt :

Bernsteinsäure, Adipinsäure, Korksäure, Azelainsäure, Sebacinsäure, Phthalsäure, Isophthalsäure, Terephthalsäure, Trimellitsäure, Phthalsäureanhydrid, Tetrahydrophthalsäureanhydrid, Hexahydrophthalsäureanhydrid, Tetrachlorphthalsäureanhydrid, Endomethylentetrahydrophthalsäureanhydrid, Glutarsäureanhydrid, Maleinsäure, Maleinsäureanhydrid, Fumarsäure, dimerisierte und trimerisierte ungesättigte Fettsäuren, gegebenenfalls in Mischung mit monomeren ungesättigten Fettsäuren, wie Ölsäure ; Terephthalsäuredimethylester und Terephthalsäure-bis-glykolester. Als mehrwertige Alkohole kommen z.B. Äthylenglykol, Propylenglykol-(1,2) und -(1,3), Butylenglykol-(1,4) und -(2,3), Hexandiol-(1,6), Octandiol-(1,8), Neopentylglykol, 1,4-Bis-Hydroxymethylcyclohexan, 2-Methyl-1,3-propandiol, Glycerin, Trimethylolpropan, Hexantriol-(1,2,6), Butantriol-(1,2,4), Trimethyloläthan, Pentaerythrit, Chinit, Mannit und Sorbit, Formit, Methylglykosid, ferner Diäthylenglykol, Triäthylenglykol, Tetraäthylenglykol und höhere Polyäthylenglykole, Dipropylenglykol und höhere Polypropylenglykole sowie Dibutylenglykol und höhere Polybutylenglykole in Frage. Die Polyester können anteilig endständige Carboxylgruppen aufweisen. Auch Polyester aus Lactonen, z.B. ε-Caprolacton, oder aus Hydroxycarbonsäuren, z.B. ω-Hydroxycapronsäure, sind einsetzbar.

b) Auch die erfindungsgemäß in Frage kommenden, mindestens zwei, in der Regel zwei bis acht, vorzugsweise zwei bis drei, Hydroxylgruppen aufweisenden Polyäther sind solche der an sich bekannten Art und werden z.B. durch Polymerisation von Epoxiden wie Äthylenoxid, Propylenoxid, Butylenoxid, Tetrahydrofuran, Styroloxid oder Epichlorhydrin mit sich selbst, z.B. in Gegenwart von Lewis-Katalysatoren wie $BF_3$, oder durch Anlagerung dieser Epoxide, vorzugsweise von Äthylenoxid und Propylenoxid, gegebenenfalls im Gemisch oder nacheinander, an Startkomponenten mit reaktionsfähigen Wasserstoffatomen wie Wasser, Alkohole, Ammoniak oder Amine, z.B. Äthylenglykol, Propylenglykol-(1,3) oder -(1,2), Trimethylolpropan, Glycerin, Sorbit, 4,4'-Dihydroxy-diphenylpropan, Anilin, Äthanolamin oder Äthylendiamin hergestellt. Auch Sucrosepolyäther, wie sie z.B. in den DE-Auslegeschriften 1 176 358 und 1 064 938 beschrieben werden, sowie auf Formit oder Formose gestartete Polyäther (DE-Offenlegungsschriften 2 639 083 bzw. 2 737 951), kommen erfindungsgemäß in Frage. Vielfach sind solche Polyäther bevorzugt, die überwiegend (bis zu 90 Gew.-%, bezogen auf alle vorhandenen OH-Gruppen im Polyäther) primäre OH-Gruppen aufweisen. Auch OH-Gruppen aufweisende Polybutadiene sind erfindungsgemäß geeignet.

c) Unter den Polythioäthern seien insbesondere die Kondensationsprodukte von Thiodiglykol mit sich selbst und/oder mit anderen Glykolen, Dicarbonsäuren, Formaldehyd, Aminocarbonsäuren oder Aminoalkoholen angeführt. Je nach den Co-Komponenten handelt es sich bei den Produkten z.B. um Polythiomischäther, Polythioätherester oder Polythioätheresteramide.

d) Als Polyacetale kommen z.B. die aus Glykolen, wie Diäthylenglykol, Triäthylenglykol, 4,4'-Dioxäthoxydiphenyldimethylmethan, Hexandiol und Formaldehyd herstellbaren Verbindungen in Frage. Auch durch Polymerisation cyclischer Acetale wie z.B. Trioxan (DE-Offenlegungschrift 1 694 128) lassen sich erfindungsgemäß geeignete Polyacetale herstellen.

e) Als Hydroxylgruppen aufweisende Polycarbonate kommen solche der an sich bekannten Art in Betracht, die z.B. durch Umsetzung von Diolen wie Propandiol-(1,3), Butandiol-(1,4) und/oder Hexandiol-(1,6), Diäthylenglykol, Triäthylenglykol, Tetraäthylenglykol oder Thiodiglykol mit Diarylcarbonaten, z.B. Diphenylcarbonat, oder Phosgen hergestellt werden können (DE-Auslegeschriften 1 694 080, 1 915 908 und 2 221 751 ; DE-Offenlegungsschrift 2 605 024).

f) Zu den Polyesteramiden und Polyamiden zählen z.B. die aus mehrwertigen gesättigten oder ungesättigten Carbonsäuren bzw. deren Anhydriden und mehrwertigen gesättigten oder ungesättigten Aminoalkoholen, Diaminen, Polyaminen und deren Mischungen gewonnenen, vorwiegend linearen Kondensate.

g) Auch bereits Urethan- oder Harnstoffgruppen enthaltende Polyhydroxylverbindungen sowie gegebenenfalls modifizierte natürliche Polyole, wie Rizinusöl oder Kohlenhydrate, z.B. Stärke, sind verwendbar. Auch Anlagerungsprodukte von Alkylenoxiden an Phenol-Formaldehyd-Harze oder auch an Harnstoff-Formaldehydharze sind erfindungsgemäß einsetzbar.

h) Die genannten Polyhydroxylverbindungen können vor ihrer Verwendung im Polyisocyanat-Polyadditionsverfahren noch in der verschiedensten Weise modifiziert werden : So läßt sich gemäß DE-Offenlegungsschriften 2 210 839 (US-Patentschrift 3 849 515) und 2 544 195 ein Gemisch aus verschiedenen Polyhydroxylverbindungen (z.B. aus einem Polyäther- und einem Polyesterpolyol) durch Verätherung in Gegenwart einer starken Säure zu einem höhermolekularen Polyol kondensieren, welches aus über Ätherbrücken verbundenen verschiedenen Segmenten aufgebaut ist.

i) Erfindungsgemäß können gegebenenfalls auch Polyhydroxylverbindungen eingesetzt werden, in welchen hochmolekulare Polyaddukte bzw. Polykondensate oder Polymerisate in feindisperser oder gelöster Form enthalten sind. Derartige Polyhydroxylverbindungen werden z.B. erhalten, wenn man Polyadditionsreaktionen (z.B. Umsetzungen zwischen Polyisocyanaten und aminofunktionellen Verbindungen) bzw. Polykondensationsreaktionen (z.B. zwischen Formaldehyd und Phenolen und/oder Aminen) in situ in den oben genannten, Hydroxylgruppen aufweisenden Verbindungen ablaufen läßt. Derartige Verfahren sind beispielsweise in den DE-Auslegeschriften 1 168 075 und 1 260 142, sowie den DE-Offenlegungsschriften 2 324 134, 2 423 984, 2 512 385, 2 513 815, 2 550 796, 2 550 797, 2 550 833, 2 550 862, 2 633 293 und 2 639 254 beschrieben. Es ist aber auch möglich, gemäß US-Patentschrift 3 869 413 bzw. DE-Offenlegungsschrift 2 550 860 eine fertige wäßrige Polymerdispersion mit einer Polyhydroxylverbindung zu vermischen und anschließend aus dem Gemisch das Wasser zu entfernen.

Auch durch Vinylpolymerisate modifizierte Polyhydroxylverbindungen, wie sie z.B. durch Polymerisation von Styrol und Acrylnitril in Gegenwart von Polyäthern (US-Patentschriften 3 383 351, 3 304 273, 3 523 093, 3 110 695 ; DE-Auslegeschrift 1 152 536) oder Polycarbonatpolyolen (DE-Patentschrift 1 769 795 ; US-Patentschrift 3 637 909) erhalten werden, sind erfindungsgemäß geeignet. Bei Verwendung von Polyätherpolyolen, welche gemäß den DE-Offenlegungsschriften 2 442 101, 2 644 922 und 2 646 141 durch Pfropfpolymerisation mit Vinylphosphonsäureestern sowie gegebenenfalls (Meth)-acrylnitril, (Meth)acrylamid oder OH-funktionellen (Meth)acrylsäureestern modifiziert wurden, erhält man Kunststoffe von besonderer Flammwidrigkeit. Polyhydroxylverbindungen, in welche durch radikalische Pfropfpolymerisation mittels ungesättigter Carbonsäuren sowie gegebenenfalls weiterer olefinisch ungesättigter Monomerer Carboxylgruppen eingeführt wurden (DE-Offenlegungsschriften 2 714 291, 2 739 620 und 2 654 746) können mit besonderem Vorteil in Kombination mit mineralischen Füllstoffen eingesetzt werden.

Bei der Verwendung von modifizierten Polyhydroxylverbindungen der oben genannten Art als Ausgangskomponente im Polyisocyanat-Polyadditionsverfahren entstehen in vielen Fällen Polyurethankunststoffe mit wesentlich verbesserten mechanischen Eigenschaften.

Vertreter der genannten erfindungsgemäß zu verwendenden Verbindungen sind z.B. in High Polymers, Vol. XVI, « Polyurethanes, Chemistry and Technology », verfaßt von Saunders-Frisch, Interscience Publishers, New York, London, Band I, 1962, Seiten 32-42 und Seiten 44-54 und Band II, 1964, Seiten 5-6 und 198-199, sowie im Kunststoff-Handbuch, Band VII, Vieweg-Höchtlen, Carl-Hanser-Verlag, München, 1966, z.B. auf den Seiten 45-71, beschrieben. Selbstverständlich können Mischungen der obengenannten Verbindungen mit mindestens zwei gegenüber Isocyanaten reaktionsfähigen Wasserstoffatomen mit einem Molekulargewicht von 400-50 000, z.B. Mischungen von Polyäthern und Polyestern, eingesetzt werden.

Von besonderem Vorteil ist es dabei in manchen Fällen, niedrigschmelzende und hochschmelzende Polyhydroxylverbindungen miteinander zu kombinieren (DE-Offenlegungsschrift 2 706 297).

Erfindungsgemäß geeignete niedermolekulare Verbindungen mit mindestens zwei gegenüber Isocyanaten reaktionsfähigen Wasserstoffatomen (Molekulargewicht von 32 bis 400) sind ebenfalls vorzugsweise Hydroxylgruppen aufweisende Verbindungen mit in der Regel 2 bis 8, vorzugsweise 2 bis 4, gegenüber Isocyanaten reaktionsfähigen Wasserstoffatomen.

Auch in diesem Fall können Mischungen von verschiedenen Verbindungen mit mindestens zwei gegenüber Isocyanaten reaktionsfähigen Wasserstoffatomen mit einem Molekulargewicht von 32 bis 400

verwendet werden.

Als Beispiele für derartige Verbindungen seien genannt:

Äthylenglykol, Propylenglykol-(1,2) und -(1,3), Butylenglykol-(1,4) und -(2,3), Pentandiol-(1,5), Hexandiol-(1,6), Octandiol-(1,8), Neopentylglykol, 1,4-Bis-hydroxymethyl-cyclohexan, 2-Methyl-1,3-propandiol, Dibrombutendiol (US-Patentschrift 3 723 392), Glyzerin, Trimethylolpropan, Hexantriol-(1,2,6), Trimethyloläthan, Pentaerythrit, Chinit, Mannit und Sorbit, Ricinusöl, Diäthylenglykol, Triäthylenglykol, Tetraäthylenglykol, höhere Polyäthylenglykole mit einem Molekulargewicht bis 400, Dipropylenglykol, höhere Polypropylenglykole mit einem Molekulargewicht bis 400, Dibutylenglykol, höhere Polybutylenglykole mit einem Molekulargewicht bis 400, 4,4'-Dihydroxy-diphenylpropan und Di-hydroxymethyl hydrochinon.

Als niedermolekulare Polyole kommen erfindungsgemäß auch die Gemische von Hydroxyaldehyden und Hydroxyketonen (« Formose ») bzw. die hieraus durch Reduktion erhaltenen mehrwertigen Alkohole (« Formit) in Frage, wie sie bei der Selbstkondensation von Formaldehydhydrat in Gegenwart von Metallverbindungen als Katalysator und von zur Endiolbildung befähigten Verbindungen als Co-Katalysator entstehen (DE-Offenlegungsschriften 2 639 084, 2 714 084, 2 714 104, 2 721 186, 2 738 154 und 2 738 512). Auch Lösungen von Polyisocyanatpolyadditionsprodukten, insbesondere von ionische Gruppen aufweisenden Polyurethanharnstoffen und/oder von Polyhydrazodicarbonamiden, in niedermolekularen, mehrwertigen Alkoholen kommen erfindungsgemäß als Polyolkomponente in Betracht (DE-Offenlegungsschrift 2 638 759).

Erfindungsgemäß geeignete Beschichtungssysteme auf Basis von Epoxyharz-Vorprodukten sind beispielsweise Triglycidylisocyanurat; Polyepoxide mit Molgewichten bis zu 2 000, wie sie aus Bisphenol A und Epichlorhydrin erhalten werden; Bisglycidylester der Terephthalsäure, der Isophthalsäure, der Phthalsäure, Tetrahydrophthalsäure, Hexahydrophthalsäure oder Hexahydroterephthalsäure; Trisglycidylester der Trimellitsäure; Tetraglycidylester und β-Methylglycidylester der Pyromellitsäure, Glycidylderivate des Hydantoins gemäß der Formel

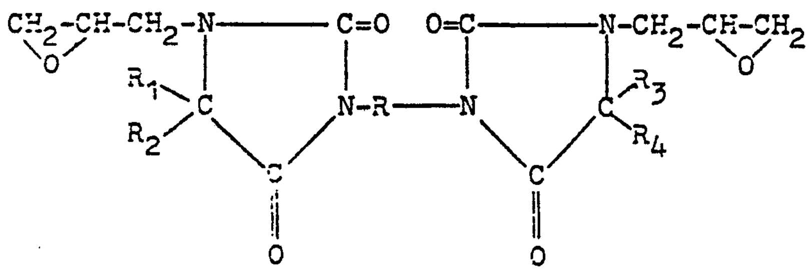

worin

R einen zweiwertigen aliphatischen, cycloaliphatischen oder araliphatischen Rest darstellt und

$R_1$, $R_2$, $R_3$ und $R_4$ je ein Wasserstoffatom oder einen aliphatischen oder cycloaliphatischen Kohlenwasserstoffrest bedeuten, oder $R_1$ und $R_2$ bzw. $R_3$ und $R_4$ zusammen einen zweiwertigen aliphatischen oder cycloaliphatischen Kohlenwasserstoffrest, vorzugsweise einen Tetramethylen- oder Pentamethylenrest, bilden.

Vorzugsweise bedeuten in der allgemeinen Formel $R_1$, $R_2$, $R_3$ und $R_4$ Wasserstoff oder niedere Alkylreste mit 1-4 C-Atomen und R einen Alkylenrest mit 1-4 C-Atomen.

Weitere Polyepoxidharze sind zugänglich durch Kondensationsreaktionen von Epichlorhydrin mit primären und/oder sekundären Aminen, Hydroxyl- und/oder Carboxylgruppen enthaltenden Polyestern, Hydroxylgruppen aufweisenden Phenol-Formaldehyd-Kondensaten oder auch Polyätherpolyolen. Die Härtung dieser Polyepoxidharze erfolgt in an sich bekannter Weise mit Aminen bzw. Polyaminen oder Amiden bzw. Polyamiden oder mit Polycarbonsäuren, wobei unter diesem Begriff z.B. auch freie Carboxylgruppen enthaltende Polyester fallen sollen, sowie auch mit Mercapto- oder phenolische Hydroxylgruppen aufweisenden Verbindungen.

Selbstverständlich können allen diesen Lacksystemen zur Erzielung spezieller Eigenschaften auch andere Harze wie z.B. Ketonharze, Nitrocellulose, PVC-Mischpolymerisate, Cellulose-acetobutyrate usw. zugemischt werden. Auch andere in der Lackiertechnik übliche Hilfsmittel wie Verlaufshilfsstoffe, Pigmente, Füllstoffe und weitere an sich bekannte Additive können erfindungsgemäß mitverwendet werden.

Die erfindungsgemäßen Beschichtungsmassen können bis zu 90 Gew.-%, vorzugsweise bis zu 60 Gew.-%, eines organischen Lösungsmittels enthalten. Beispiele für solche Lösungsmittel sind Toluol, Xylol, aromatenarme bzw. aromatenfreie Kohlenwasserstofffraktionen, Äthylacetat, Butylacetat, Äthylenglykolmonomethylätheracetat, Äthylenglykolmonoäthylätheracetat, Aceton, Methyläthylketon, Cyclohexanon sowie Mischungen dieser Verbindungen.

Die erfindungsgemäßen Beschichtungsmassen eignen sich für die Lackierung bzw. Beschichtung beliebiger Unterlagen wie z.B. von Metallen wie Aluminium oder Stahl, von Asbestzement, Leder, Textilien, Gummi, Papier, Glas, Stein und den verschiedenartigsten Kunststoffen. Besonders geeignet sind sie für die Lackierung von Holz und Metall.

Bevorzugt werden die erfindungsgemäßen Lack- und Beschichtungssysteme maschinell verarbeitet.

Man bedient sich dabei der an sich bekannten Auftragstechniken wie z.B. Spritzen, Sprühen, Tauchen, Rollen und Gießen. Die Schichtdicken liegen im allgemeinen zwischen 1 und 1 000 μ, vorzugsweise zwischen 4 und 200 μ.

Nach dem Auftragen des Lacks bzw. der Beschichtung wird mit Licht der Wellenlänge 250 bis 500 nm belichtet. Die Bestrahlungsdauer beträgt im allgemeinen 0,1 bis 300 Sekunden, vorzugsweise 1 bis 80 Sekunden, je nach der Dicke der aufgetragenen Schicht und der Leistung des Strahlers. Unter den Einfluß des kurzwelligen Lichts zerfällt, wie schon erläutert, das im Lack enthaltene erfindungsgemäße Ammoniumsalz unter Decarboxylierung. Das freiwerdende Amin wirkt dann als Katalysator für die Härtungsreaktion bzw. — im Falle des Ammoniumsalzes eines primären oder sekundären Polyamins — in an sich bekannter Weise als Vernetzungsmittel, z.B. für ein Vorpolymeres mit freien NCO-Gruppen.

Vorzugsweise wird erfindungsgemäß etwa bei Raumtemperatur (ca. 10-30 °C) beschichtet. Da die erfindungsgemäßen Ammoniumsalze sich beim Erhitzen auch thermisch zersetzen, läßt sich durch Erwärmen (bis ca. 130 °C, vorzugsweise bis 80 °C) eine weitere Beschleunigung der Härtungsreaktion erreichen. Der Vorteil der erfindungsgemäßen Harzkompositionen liegt jedoch darin, daß sie auch bei niedrigen Temperaturen, d.h. ohne jede thermische Belastung des Substrats, verarbeitet werden können.

Die erfindungsgemäßen Beschichtungsmassen haben bei Raumtemperatur eine überraschend große Lagerbeständigkeit, d.h. eine lange Verarbeitbarkeit ; bei der Bestrahlung mit kurzwelligem Licht gelieren sie jedoch außerordentlich rasch und härten danach schnell aus — überraschenderweise auch dann, wenn es sich um keine Klarlacke sondern um pigmentierte Systeme handelt.

Ein besonderer Vorteil der erfindungsgemäßen Initiatoren ist, daß sie zu keiner Verfärbung des Lackes bei der Belichtung oder bei Wärmeeinwirkung führen.

Die folgenden Beispiele dienen zur Erläuterung der Erfindung. Wenn nicht anders vermerkt, sind Mengenangaben als Gewichtsteile bzw. Gewichtsprozente zu verstehen.

## Beispiel 1

Zu einer Lösung von 8,8 g Brenztraubensäure in 50 ml Äther wird eine Lösung von 11 g Diazabicyclooctan (DABCO(R)) in 200 ml Äther zugetropft. Man erhält 13,5 g (67 % der Theorie) des Mono-ammoniumsalzes von Diazabicyclooctan in kristalliner Form.

## Beispiel 2

Zu einer Lösung von 13 g 3,3-Dimethyl-2-oxo-buttersäure in 100 ml Äther wird eine Lösung von 7,3 g Diäthylamin in 50 ml Äther zugetropft. Man erhält 16,7 g (82 % der Theorie) des Diäthylammoniumsalzes in kristalliner Form. Schmp. : 164 °C (Zers.)

## Beispiel 3

Zu einer Lösung von 15 g Phenylglyoxylsäure in 100 ml Äther wird eine Lösung von 14,9 g Triäthanolamin in 100 ml Äther zugetropft. Man erhält 28,1 g (94 % der Theorie) des Triäthanolammoniumsalzes in kristalliner Form.

## Beispiel 4 bis 9

a) Herstellung substituierter Phenylglyoxylsäuren :

Literatur :

K. Kindler et al., Chem. Ber. *76,* 308 (1943)

40 g $AlCl_3$ werden bei Raumtemperatur in 100-150 ml Nitrobenzol gelöst, 0,2 mol Äthoxalylchlorid unter Kühlung zugegeben und bei 10 °C 0,2 mol des substituierten Aromaten zugetropft. Man läßt 5 Stunden bei Raumtemperatur nachrühren, gießt auf 500 g Eis und 100 ml HCl, äthert aus und trocknet die organische Phase. Nach dem Abziehen des Lösungsmittels am Rotationsverdampfer wird der Rückstand destilliert.

Das Destillat, bestehend aus dem substituierten Phenylglyoxylsäureäthylester, wird in warme 10 %ige NaOH gegossen, 30 Minuten bei 40 °C nachgerührt, angesäuert, ausgeäthert und die Ätherphase getrocknet. Nach dem Abziehen des Lösungsmittels wird der Rückstand umkristallisiert.

Die wie eben beschrieben hergestellten Ester bzw. Säuren der allgemeinen Formel

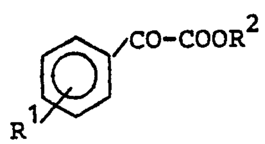

haben die in der nachstehenden Tabelle angegebenen Eigenschaften (Ø steht für einen Phenylrest) :

| $R^1$ | $R^2$ | $K_{p(Torr)}$ | Schmelzpunkt (°C) |
|---|---|---|---|
| 4-$CH_3$ | $C_2H_5$ | $90_{(0,1)}$ | - |
| 4-$CH_3$ | H | – | 92-95 |
| 4-$CH_2$-$CH_3$ | $C_2H_5$ | $110_{(0,4)}$ | - |
| 4-$CH_2$-$CH_3$ | H | – | 65-68 |
| 4-$OCH_3$ | $C_2H_5$ | $130_{(0,4)}$ | - |
| 4-$OCH_3$ | H | – | 91-92 |
| 4-Ø | $C_2H_5$ | $185_{(0,4)}$ | - |
| 4-Ø | H | – | 106-108 |
| 3.4-$OCH_3$ | $C_2H_5$ | $148_{(0,3)}$ | - |
| 3.4-$OCH_3$ | H | – | 136-137 |

In analoger Weise wurden auch der Äthylester von α-Naphthylglyoxylsäure bzw. die freie Säure hergestellt.

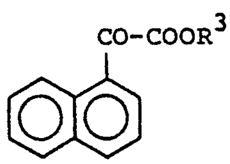

| | | $K_{p(Torr)}$ | Schmelzpunkt (°C) |
|---|---|---|---|
| | $R^3 = C_2H_5$ | $152_{(0,5)}$ | — |
| | H | — | 111 |

b) Herstellung der erfindungsgemäßen Ammoniumsalze :

Zu einer Lösung von 0,1 mol der substituierten Phenylglyoxylsäure in Diäthyläther wird eine Lösung von 0,1 mol Dimethylbenzylamin in Diäthyläther zugetropft. Man erhält die Ammoniumsalze der allgemeinen Formeln

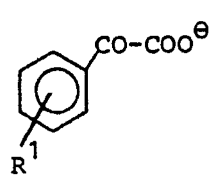

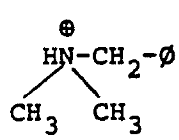

bzw.

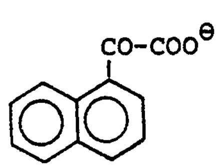

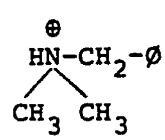

in kristalliner Form.

| Beispiel | $R^1$ | Schmelzpunkt |
|---|---|---|
| 4 | 4-$CH_3$ | 115-120 |
| 5 | 4-$CH_2$-$CH_3$ | 55-64 |
| 6 | 4-$OCH_3$ | 73-81 |
| 7 | 4-∅ | 106-110 |
| 8 | 3.4-$OCH_3$ | 58-62 |
| 9 | Ammoniumsalz der α-Naphthylglyoxylsäure | 114-121 |

Beispiel 10

UV-härtender Polyurethanlack

Ein Klarlack, bestehend aus

154 Teilen eines Polyesters aus 1 Mol Phthalsäureanhydrid, 2 Mol Hexahydrophthalsäureanhydrid, 3,45 Mol Trimethylolpropan und 1 Mol Maleinsäureanhydrid, 67 %ig gelöst in Äthylenglykolmonoäthylätheracetat/Xylol (1 : 1),

99 Teilen Lösungsmittel S,

3,8 Teilen einer 10 %igen Lösung von Celluloseacetobutyrat in Lösungsmittel S,

120 Teilen einer 75 %igen Lösung eines biuretgruppenhaltigen aliphatischen Polyisocyanats (Umsetzungsprodukt aus 3 Mol Hexamethylendiisocyanat und 1 Mol Wasser) in Äthylenglykolmonoäthylätheracetat/Xylol (1 :1),

der unterschiedliche Mengen an den Initiatoren A bzw. B enthielt, wurde in einer Schichtdicke von 15 μ auf eine Glasplatte aufgebracht.

In einer zweiten Versuchsreihe wurde ein pigmentierter Lack, der wie der Klarlack zusammengesetzt war, jedoch 222 Teile Lösungsmittel S und zusätzlich 114 Teile eines Titandioxid-Weißpigments enthielt, ebenfalls in einer Schichtdicke von 15 μ auf eine Glasplatte aufgetragen.

Die Lackfilme wurden in 4 cm Abstand von einer UV-Lampe der Leistung 1KW und des Intensitätsmaximumms bei ca. 300-320 nm (Typ HT Q-4-Strahler der Firma Philips) 60 Sekunden lang bestrahlt. Die Initiator enthaltenden Lacke (sowohl der Klarlack als auch der pigmentierte Lack) waren danach grifftrocken ; die Lackierungen ohne Initiator waren hingegen noch stark klebrig.

Nach einer Lagerung von 1 Tag bei Raumtemperatur nach der Belichtung wurde die Härte der Lackfilme nach König bestimmt (DIN 53 157).

Die Ergebnisse sind in der folgenden Tabelle zusammengefaßt. Die Mengenangabe des Initiators ist bezogen auf Feststoffgehalt des Lacks.

| | kein Initiator | Initiator A | | | Initiator B | | |
|---|---|---|---|---|---|---|---|
| | | 3% | 4% | 5% | 3% | 4% | 5% |
| Klarlack | nicht meßbar | 182 | 192 | 197 | 195 | 185 | 132 |
| pigmentierter Lack | 150 | 185 | 185 | 188 | 193 | 192 | 200 |

Lösungsmittel S ist ein Gemisch aus gleichen Teilen an Methyläthylketon, Toluol, Butylacetat und Äthylenglykolmonoäthylätheracetat ;

Initiator A ist das Dimethylbenzylammoniumsalz von Phenylglyoxylsäure ;

Initiator B ist das Tri-n-butylammoniumsalz von Phenylglyoxylsäure.

Beispiel 11

Lacke, bestehend aus
264 Teilen eines pigmentierten Polyols,
100 Teilen derselben Polyisocyanatlösung wie in Beispiel 10 und
7 Teilen Initiator
wurden in einer Schichtdicke von 15 μ auf Glasplatten aufgetragen und, wie in Beispiel 10 beschrieben, belichtet, 1 Tag gelagert und auf ihre Härte untersucht. Die Ergebnisse sind in der folgenden Tabelle zusammengestellt:

| Initiator<br>Dimethylbenzylammoniumsalz von | Pendelhärte (sec.) |
|---|---|
| Phenylglyoxylsäure | 177 |
| p-Methylphenylglyoxylsäure | 171 |
| p-Äthylphenylglyoxylsäure | 115 |
| p-Methoxyphenylglyoxylsäure | 112 |
| 3,4-Dimethoxyphenylglyoxylsäure | 98 |
| p-Phenylphenylglyoxylsäure | 149 |
| α-Naphthylglyoxylsäure | 171 |
| ohne Initiator | nicht meßbar |

Das pigmentierte Polyol besteht aus
100 Teilen eines Rutil-Titandioxidpigments und
164 Teilen eines Polyesters (8 % OH-Gruppen; Säurezahl 4) aus 3 Mol Phthalsäureanhydrid, 0,05 Mol Maleinsäureanhydrid und 3,5 Mol Trimethylolpropan, 61 %ig gelöst in Äthylenglykolmonoäthylätheracetat.

Beispiel 12

Ein Lack, bestehend aus
264 Teilen des pigmentierten Polyols aus Beispiel 11,
100 Teilen derselben Isocyanatlösung wie in Beispiel 10 und
0,5 Teilen des Ammoniumsalzes aus einem Mol Diazabicycloundecen und einem Mol Phenylglyoxylsäure wird in einer Schichtstärke von 60 μ auf Glasplatten aufgetragen und 15 Minuten bei 80 °C eingebrannt. Nach dieser Zeit ist der Film trocken, während ein Film ohne Katalysator noch klebrig ist.

**Ansprüche**

1. Ammoniumsalze auf Basis von Aminen eines zwischen 31 und 500 liegenden Molekulargewichts und von α-Ketocarbonsäuren der allgemeinen Formel

$$A—CO—COOH \qquad (I)$$

in welcher

A für Wasserstoff, eine Hydroxyl- oder Carboxylgruppe, —COR, —CN, einen gegebenenfalls verzweigten Alkylrest mit 1 bis 6 C-Atomen, welcher gegebenenfalls durch Halogen, —OH, —COOH, —COOR, —CN, —OR, —COR oder —COR' substituiert ist, einen Cycloalkylrest mit 4 bis 10 C-Atomen, einen Aryl- oder Aralkylrest mit 6 bis 15 C-Atomen, welcher gegebenenfalls durch —OH, —R, OR, —SR, Halogen, —$NO_2$, —COR, —COOH, —CN, —COOR, —$CONH_2$, —OR', —SR' oder —COR' substituiert ist, oder einen Sauerstoff und/oder Stickstoff als Heteroatom enthaltenden heterocyclischen Rest mit 4 bis 10 C-Atomen darstellt, wobei

R für eine gegebenenfalls halogensubstituierte Alkylgruppe mit 1 bis 6 C-Atomen und
R' für eine Arylgruppe mit 6 bis 12 C-Atomen stehen,

wobei das Salz auf Basis von Triäthanolamin und Brenztraubensäure ausgenommen ist.

2. Ammoniumsalze nach Anspruch 1, dadurch gekennzeichnet, daß A für einen gegebenenfalls durch Halogen, eine Methyl-, Methoxy- oder Phenylgruppe substituierten Phenylrest steht.

3. Ammoniumsalze nach Anspruch 1, dadurch gekennzeichnet, daß die Aminkomponente ein Molekulargewicht von 100 bis 300 aufweist.

4. Ammoniumsalze nach Anspruch 1 bis 3, dadurch gekennzeichnet, daß die Aminkomponente 1 bis 5 Aminstickstoffatome enthält.

5. Ammoniumsalze nach Anspruch 1 bis 4, dadurch gekennzeichnet, daß die Aminkomponente ein tertiäres Amin ist.

6. Verwendung der Ammoniumsalze gemäß Anspruch 1 bis 5 zur Herstellung der ihnen zugrundeliegenden Amine, dadurch gekennzeichnet, daß man die Ammoniumsalze mit Licht einer Wellenlänge zwischen 250 und 500 nm bestrahlt und/oder auf 50-130 °C, vorzugsweise 50-80 °C erhitzt.

7. Beschichtungsmassen auf Basis von in Gegenwart von Aminen aushärtenden Polyurethan- oder Epoxyharzvorprodukten, dadurch gekennzeichnet, daß sie 0,1 bis 40 Gew.-%, bezogen auf Feststoffgehalt, eines Ammoniumsalzes gemäß Anspruch 1 bis 5 enthalten.

8. Beschichtungsmassen nach Anspruch 7, dadurch gekennzeichnet, daß sie 3 bis 10 Gew.-%, bezogen auf Ammoniumsalz, eines Sensibilisators enthalten.

## Claims

1. Ammonium salts based on amines having a molecular weight of between 31 and 500 and on α-ketocarboxylic acids of the general formula

$$A-CO-COOH \qquad (I)$$

wherein

A represents hydrogen, a hydroxyl or carboxyl group, —COR, —CN, an optionally branched alkyl radical containing from 1 to 6 carbon atoms which may optionally be substituted by halogen, —OH, —COOH, —COOR, —CN, —OR, —COR, or COR′, a cycloalkyl radical containing from 4 to 10 carbon atoms, an aryl or aralkyl radical containing from 6 to 15 carbon atoms which may optionally be substituted by —OH, —R, —OR, —SR, halogen, $—NO_2$, —COR, —COOH, —CN, —COOR, $—CONH_2$, —OR′, —SR′ or —COR′, or a $C_4$-$C_{10}$-heterocyclic radical containing oxygen and/or nitrogen as a hetero atom,

wherein

R represents an optionally halogen-substituted alkyl group containing from 1 to 6 carbon atoms and
R′ represents an aryl group containing from 6 to 12 carbon atoms,

the salt based on triethanolamine and pyruvic acid being excluded.

2. Ammonium salts according to Claim 1, characterised in that A represents a phenyl radical optionally substituted by halogen, a methyl, methoxy or phenyl group.

3. Ammonium salts according to Claim 1, characterised in that the amine component has a molecular weight of from 100 to 300.

4. Ammonium salts according to Claim 1 to 3, characterised in that the amine component contains from 1 to 5 amine nitrogen atoms.

5. Ammonium salts according to Claim 1 to 4, characterised in that amine component is a tertiary amine.

6. Use of the ammonium salts according to Claim 1 to 5 for producing the amines on which they are based, characterised in that the ammonium salts are irradiated with light having a wavelength of from 250 to 500 nm and/or are heated to 50-130 °C, preferably 50-80 °C.

7. Coating compositions based on polyurethane or epoxy resin precursors hardening in the presence of amines, characterised in that they contain from 0.1 to 40 % by weight, based on solids content, of an ammonium salt according to Claim 1 to 5.

8. Coating compositions according to Claim 7, characterised in that they contain from 3 to 10 % by weight, based on ammonium salt, of a sensitiser.

## Revendications

1. Sels d'ammonium à base d'amines d'un poids moléculaire se situant entre 31 et 500 et d'acides α-cétocarboxyliques de formule générale

$$A-CO-COOH \qquad (I)$$

dans laquelle

A représente l'hydrogène, un groupe hydroxy ou carboxy, —COR, —CN, un reste alkyle en $C_1$-$C_6$

éventuellement ramifié, qui est éventuellement substitué par un halogène, —OH, —COOH, —COOR, —CN, —OR, —COR ou COR', un reste cycloalkyle en $C_4$-$C_{10}$, un reste aryle ou arylalkyle en $C_6$-$C_{15}$, qui est éventuellement substitué par —OH, —R, —OR, —SR, un halogène, —$NO_2$, —COR, —COOH, —CN, —COOR, —$CONH_2$, —OR', —SR' ou —COR', ou bien un reste hétérocyclique en $C_4$-$C_{10}$ contenant un atome d'oxygène et/ou un atome d'azote comme hétéroatome,

R représente un groupe alkyle en $C_1$-$C_6$ éventuellement halogéné, et R' représente un groupe aryle en $C_6$-$C_{12}$, à l'exception du sel à base de triéthanolamine et de l'acide pyrotartrique.

2. Sels d'ammonium selon la revendication 1, caractérisés en ce que A représente un reste phényle éventuellement substitué par un halogène ou un groupe méthyle, méthoxy ou phényle.

3. Sels d'ammonium selon la revendication 1, caractérisés en ce que le composant amine a un poids moléculaire de 100 à 300.

4. Sels d'ammonium selon les revendications 1 à 3, caractérisés en ce que le composant amine contient 1 à 5 atomes d'azote de groupes amino.

5. Sels d'ammonium selon les revendications 1 à 4, caractérisés en ce que le composant amine est une amine tertiaire.

6. Utilisation des sels d'ammonium selon les revendications 1 à 5 pour la préparation des amines dont ils dérivent, caractérisée en ce que l'on irradie les sels d'ammonium avec de la lumière d'une longueur d'ondes entre 250 et 500 nm et/ou on chauffe à 50-130 °C, de préférence 50-80 °C.

7. Mélanges d'enduction à base de précurseurs de résines de polyuréthanne ou de résines époxy durcissant en présence d'amines, caractérisés en ce qu'ils contiennent 0,1 à 40 % en poids, par rapport à la teneur en matière solide, d'un sel d'ammonium selon les revendications 1 à 5.

8. Mélanges d'enduction selon la revendication 7, caractérisés en ce qu'ils contiennent 3 à 10 % en poids, par rapport au sel d'ammonium, d'un sensibilisateur.